# EUROPEAN PATENT APPLICATION

(11) **EP 4 837 902 A1**
(43) Date of publication of application: **11.11.2026**
(21) Application number: 25736226.9
(22) Date of filing: 03.01.2025
(51) Int. Cl.: C07D 317/28, A61K 47/18

(54) **LIPID COMPOUND AND LIPID NANOPARTICLE FOR DELIVERY**

(30) Priority: 05.01.2024 CN 202410017912
(71) Applicant: Rinuagene Biotechnology Co., Ltd., Suzhou, Jiangsu 215000 (CN); Rinuagene International HK Limited, Kowloon Hong Kong 999077 (HK)
(72) Inventor: DONG, Yijie, (Jiangsu) Pilot Free Trade Zone Suzhou, Jiangsu 215000 (CN); LV, Kai, (Jiangsu) Pilot Free Trade Zone Suzhou, Jiangsu 215000 (CN)
(74) Representative: Chung, Hoi Kan
(86) International application number: PCT/CN2025/070378
(87) International publication number: WO 2025/146125

(57) **Abstract**

The present application discloses a compound with a structural formula shown as formula (I), and a salt and an isomer thereof. The present application further discloses a nanoparticle composition including the compound, or the salt or the isomer thereof. Nanoparticles of the present application may efficiently deliver a drug or a vaccine into a cell, to exert a therapeutic or prophylactic effect of the drug or the vaccine.

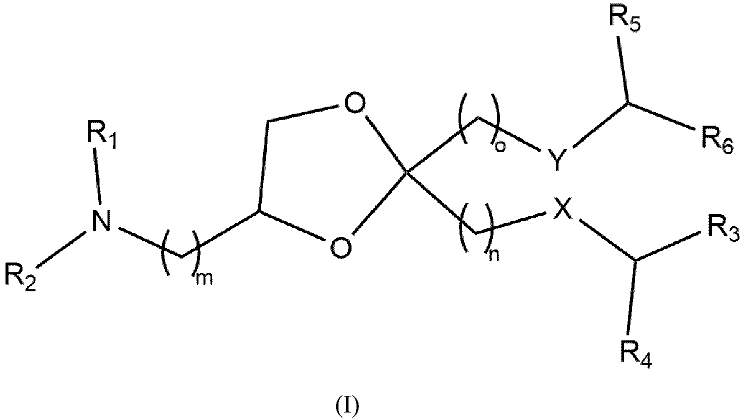

## Description

### TECHNICAL FIELD

The present application relates to the field of biotechnology, and in particular to a lipid compound and a lipid nanoparticle composition for delivery of an effective component such as a drug and a vaccine.

### BACKGROUND

Lipid nanoparticles (LNPs) have drawn widespread attention for their favorable in vitro stability, in vivo degradability, safety, and reliability, and have been widely used in mRNA vaccine research.

Lipid containing nanoparticles, or lipid nanoparticles, liposomes, and lipid complexes have been demonstrated as effective delivery carriers for bioactive substances that enter cells and/or act intracellularly, such as small molecule drugs, proteins, and nucleic acids. LNPs are small vesicles composed of one or more lipid components, and can effectively compact and deliver various nucleic acid molecules, from DNA and RNA to chromosomes, and even cells. The well-defined formulation and facile modification with targeting ligands make LNPs amenable to large-scale production.

The Onpattro^{™} LNP formulation includes four main lipid components, namely ionizable amino lipids, distearoyl phosphatidylcholine (DSPC), cholesterol, and polyethylene glycol-conjugated lipids (PEG-lipids), at a molar ratio of 50/10/38.5/1.5, respectively. Onpattro^{™} is still considered as the gold standard for benchmarking LNP-mediated efficacy studies.

Therefore, there remains a need in the art for improved in vivo expression of nucleic acids (e.g., mRNA) from lipid nanoparticles.

### SUMMARY

In view of the above, the present application discloses a lipid compound and a lipid nanoparticle composition including the compound, where the lipid has advantages such as a high encapsulation efficiency and a high expression level.

Specifically, the following technical solutions are adopted in the present application.
1. A compound of formula (I), or a salt or an isomer thereof, 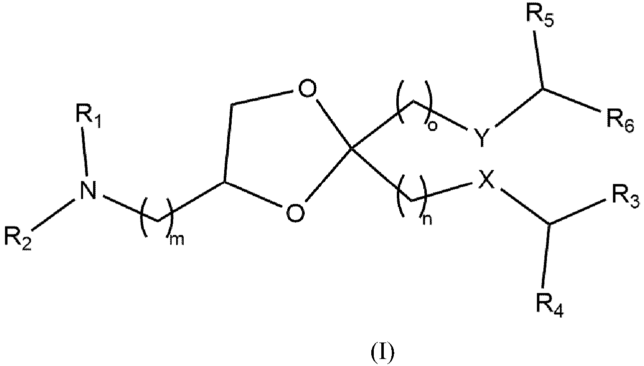
   where R1 and R2 are independently C1-C6 alkyl groups; R3 and R4 are independently C1-C12 alkyl groups; R5 and R6 are independently selected from hydrogen or C1-C12 alkyl groups;
   X and Y are independently selected from (C=O)O or O(C=O); and
   m, n, and o are independently selected from 1, 2, 3, 4, 5, 6, 7, 8, 9, and 10.
2. The compound, or the salt or the isomer thereof according to item 1, where R1 and R2 are each independently a C1-C3 alkyl group, preferably a methyl group.
3. The compound, or the salt or the isomer thereof according to item 1 or 2, where R3 and R4 are each independently a C1-C12 linear alkyl group.
4. The compound, or the salt or the isomer thereof according to any one of items 1 to 3, where R3 and R4 are each independently a C5-C9 linear alkyl group, preferably a C6-C8 linear alkyl group, more preferably a C6 or C8 linear alkyl group.
5. The compound, or the salt or the isomer thereof according to any one of items 1 to 4, where R5 is hydrogen, and R6 is C1-C12 n-alkane.
6. The compound, or the salt or the isomer thereof according to item 5, where R5 is hydrogen, and R6 is C8-C11 n-alkane, preferably C10 n-alkane.
7. The compound, or the salt or the isomer thereof according to any one of items 1 to 4, where R5 and R6 are each independently a C1-C12 linear alkyl group.
8. The compound, or the salt or the isomer thereof according to item 7, where R5 and R6 are each independently a C5-C9 linear alkyl group, preferably a C6-C8 linear alkyl group.
9. The compound, or the salt or the isomer thereof according to any one of items 1 to 8, where X is (C=O)O and Y is O(C=O), or X is (C=O)O and Y is (C=O)O, or X is O(C=O) and Y is O(C=O), or X is O(C=O) and Y is (C=O)O.
10. The compound, or the salt or the isomer thereof according to any one of items 1 to 9, where m is selected from 3, 4, 5, 6, and 7, preferably 4, 5, and 6.
11. The compound, or the salt or the isomer thereof according to any one of items 1 to 10, where n is selected from 4, 5, 6, 7, 8, and 9, preferably 5, 6, 7, and 8.
12. The compound, or the salt or the isomer thereof according to any one of items 1 to 11, where o is selected from 4, 5, 6, 7, 8, and 9, preferably 5, 6, 7, and 8.
13. The compound according to item 1, where the compound of (I) is selected from: 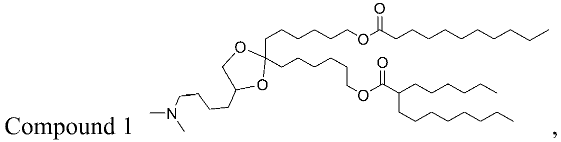 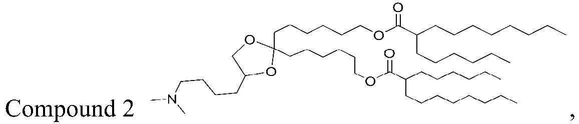 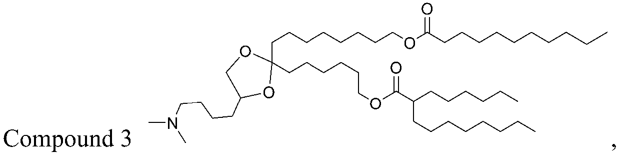 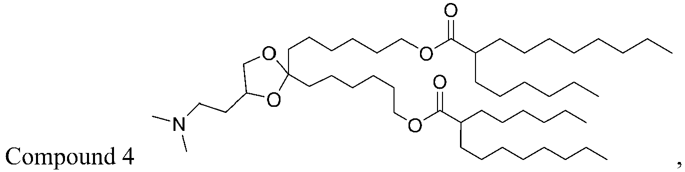 and 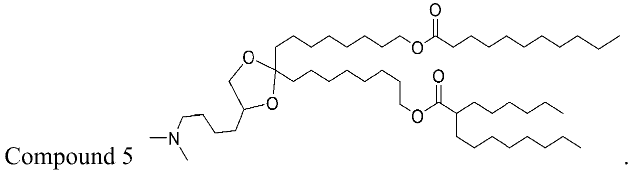
14. A lipid nanoparticle composition, including a lipid component, the lipid component including the compound according to any one of items 1 to 13.
15. The lipid nanoparticle composition according to item 14, where the lipid component further includes a phospholipid.
16. The lipid nanoparticle composition according to item 15, where the phospholipid is selected from one or more of the following compounds:
   dilauroyl phosphatidylcholine (DLPC),
   dimyristoyl phosphatidylcholine (DMPC),
   dioleoyl phosphatidylcholine (DOPC),
   dipalmitoyl phosphatidylcholine (DPPC),
   distearoyl phosphatidylcholine (DSPC),
   dioleoyl phosphatidylcholine (DUPC),
   palmitoyl oleoyl phosphatidylcholine (POPC),
   1,2-di-O-octadecyl-sn-glycero-3-phosphocholine (18:0 Diether PC), 1- oleoyl-2-cholesterylhemisuccinoyl-sn-glycero-3-phosphocholine (OChemsPC),
   1-hexadecyl-sn-glycero-3-phosphocholine (C16 Lyso PC),
   1,2-divinyl-sn-glycero-3-phosphocholine,
   1,2-diarylacyl-sn-glycero-3-phosphocholine,
   1,2-dioleoyl-SN-glycero-3-phosphoethanolamine (DOPE),
   1,2-dihydroxytin-sn-glycero-3-phosphoethanolamine (ME 16.0 PE),
   1,2-distearoyl-sn-glycero-3-phosphoethanolamine,
   1,2-divinylol-sn-glycero-3-phosphoethanolamine,
   1,2-divinyl-sn-glycero-3-phosphoethanolamine,
   1,2-diaryl-sn-glycero-3-phosphoethanolamine,
   1,2-dithiohexaenoyl-sn-glycero-3-phosphoethanolamine, and
   1,2- diolyl -sn-glycero-3-phosphatidyl-(1-glycerol) sodium salt (DOPG) or sphingomyelin.
17. The nanoparticle composition according to item 15, where the phospholipid is DOPE.
18. The nanoparticle composition according to item 15, where the phospholipid is DSPC.
19. The nanoparticle composition according to any one of items 14 to 18, where the lipid component further includes a structural lipid.
20. The nanoparticle composition according to item 19, where the structural lipid is selected from one or more of cholesterol, coprostanol, sitosterol, ergosterol, and stigmasterol.
21. The nanoparticle composition according to any one of item 19, where the structural lipid is cholesterol.
22. The nanoparticle composition according to any one of items 14 to 21, where the lipid component further includes a PEG lipid.
23. The nanoparticle composition according to item 22, where the PEG lipid is selected from one or more of PEG-modified phosphatidylethanolamine, PEG-modified phosphatidic acid, PEG-modified ceramide, PEG-modified dialkylamine, PEG-modified diacylglycerol, or PEG-modified dialkylglycerol.
24. The nanoparticle composition according to any one of items 14 to 23, where the lipid component further includes a cationic lipid and/or an ionizable lipid.
25. The nanoparticle composition according to any one of items 14 to 24, further including a therapeutic agent and/or a prophylactic agent, where the therapeutic agent and/or the prophylactic agent is selected from a vaccine, or a compound and a nucleic acid enabling triggering of an immune response, and
   preferably, the nucleic acid is RNA selected from one or more of siRNA, aiRNA, miRNA, dsRNA, shRNA, or mRNA.
26. The nanoparticle composition according to any one of items 14 to 25, where an encapsulation efficiency of the therapeutic agent and/or the prophylactic agent is greater than or equal to 50%; or greater than or equal to 80%; or greater than or equal to 90%.
27. The nanoparticle composition according to any one of items 14 to 26, where an average particle size of the nanoparticle composition is 60-130 nm.
28. The nanoparticle composition according to any one of items 14 to 27, where a polydispersity index of the nanoparticle composition is 0.04-0.20.
29. Use of the compound according to any one of items 1 to 13 in preparation of a lipid nanoparticle composition.
30. A pharmaceutical composition, including the nanoparticle composition according to any one of items 14 to 28 and a pharmaceutically acceptable carrier.
31. A method for delivering a therapeutic agent and/or a prophylactic agent to a mammalian cell, including administering the nanoparticle composition according to any one of items 14 to 28 or the pharmaceutical composition according to item 30 to a subject, the administering including bringing a cell into contact with the nanoparticle composition or the pharmaceutical composition to deliver the therapeutic agent and/or the prophylactic agent to the cell.
32. The method according to item 31, where the mammalian cell is in a mammal.
33. The method according to item 31 or 32, where the mammal is a human.
34. The method according to any one of items 31 to 33, where the nanoparticle composition is administered intravenously, intramuscularly, intradermally, subcutaneously, intranasally, or by inhalation.
35. A method for producing a polypeptide of interest in a mammalian cell, the method including bringing a cell into contact with the nanoparticle composition according to any one of items 14 to 28 or the pharmaceutical composition according to item 30 to deliver a therapeutic agent and/or a prophylactic agent to the cell, where the therapeutic agent and/or the prophylactic agent is mRNA that encodes the polypeptide of interest, and the mRNA is translated in the cell to produce the polypeptide of interest.
36. The method according to item 35, where the mammalian cell is in a mammal.
37. The method according to item 35 or 36, where the mammalian cell is a human.
38. The method according to any one of items 35 to 37, where the nanoparticle composition or the pharmaceutical composition is administered intravenously, intramuscularly, intradermally, subcutaneously, intranasally, or by inhalation.
39. A method for treating diseases or disorders in mammals, including administering a therapeutically effective amount of the nanoparticle composition according to any one of items 14 to 28 or the pharmaceutical composition according to item 30 to a mammal.
40. The method according to item 39, where the diseases or disorders are characterized by dysfunctional or abnormal protein or polypeptide activity.
41. The method according to item 39 or 40, where the diseases or disorders are selected from infectious diseases, cancer and proliferative diseases, genetic diseases, autoimmune diseases, diabetes, neurodegenerative diseases, cardiovascular diseases, renal vascular diseases, or metabolic diseases.
42. The method according to any one of items 39 to 41, where the mammal is a human.
43. The method according to any one of items 39 to 42, where the nanoparticle composition or the pharmaceutical composition is administered intravenously, intramuscularly, intradermally, subcutaneously, intranasally, or by inhalation.
44. A method for specifically delivering a therapeutic agent and/or a prophylactic agent to an organ of a mammal, including administering the nanoparticle composition according to any one of items 14 to 28 or the pharmaceutical composition according to item 30 to the mammal, the administering including bringing the organ of the mammal into contact with the nanoparticle composition to deliver the therapeutic agent and/or the prophylactic agent to the organ.
45. The method according to item 44, where the mammal is a human.
46. The method according to item 44 or 45, where the nanoparticle composition is administered intravenously, intramuscularly, intradermally, subcutaneously, intranasally, or by inhalation.
47. The method according to any one of items 44 to 46, where the mammal is pretreated 24 hours or less before the contact or administration step.
48. The method according to any one of items 44 to 47, where the mammal is pretreated about 1 hour before the contact or administration step.

### Effects of the invention

The compound of the present application may be used for preparation of lipid nanoparticles, and the nanoparticle composition containing the compound provided in the present application may achieve encapsulation and delivery of therapeutic/prophylactic agents, safely deliver the therapeutic/prophylactic agents to a target site, achieve high expression, and exert the effects of the therapeutic/prophylactic agents.

The lipid nanoparticles prepared in the present application have a small average particle size, high encapsulation efficiency, and high expression, and have broad application prospects in the field of drug delivery.

### BRIEF DESCRIPTION OF DRAWINGS

In order to provide a clearer description of the technical solutions in specific embodiments of the present invention or the prior art, the following briefly introduces the accompanying drawings required for describing the specific embodiments or the prior art.

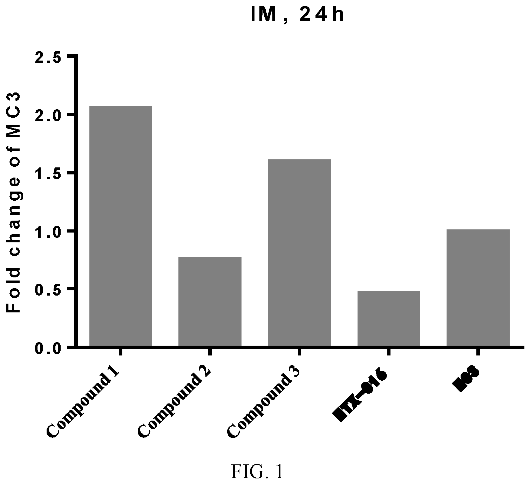

FIG. 1 shows the luciferase fluorescence intensity displayed 24 hours after intramuscular injection of different LNP formulations.

### DETAILED DESCRIPTION OF THE EMBODIMENTS

Exemplary examples of the present application are described below, and various details of the examples of the present application are included to facilitate understanding and should be considered exemplary only. Therefore, a person of ordinary skill in the art should be aware that a variety of changes and modifications can be made to the examples described here without departing from the scope and spirit of the present application. Moreover, for clarity and conciseness, descriptions of well-known functions and structures are omitted in the following description.

### Terms and definitions

As used herein, the term "alkyl group" refers to an alkyl group containing one or more carbon atoms (e.g., 1, 2, 3, 4, 5, 6, 7, 8, 9, 10, 11, 12, 13, 14, 15, 16, 17, 18, 19, 20, or more carbon atoms), which is optionally substituted. The term "C1-C12 alkyl group" refers to an optionally substituted, linear or branched, saturated hydrocarbon containing 1 to 12 carbon atoms. Unless otherwise specified, an alkyl group described herein refers to an unsubstituted and substituted alkyl group.

Unless otherwise specified, an alkyl group may be optionally substituted. Optional substituents may be selected from, but are not limited to, halogen atoms (e.g., chloro, bromo, fluoro, or iodo groups), carboxylic acids (e.g., -C(O)OH), alcohols (e.g., hydroxy group, -OH), esters (e.g., -C(O)OR or -OC(O)R), aldehydes (e.g., -C(O)H), carbonyl groups (e.g., -C(O)R, or represented by C=O), acyl halides (e.g., -C(O)X, where X is a halide selected from bromides, fluorides, chlorides, and iodides), carbonates (e.g., -OC(O)OR), alkoxy groups (e.g., -OR), acetals, phosphates, thiols (e.g., -SH), sulfoxides (e.g., - S(O)R), sulfites (e.g., -S(O)OH), sulfonic acid (e.g., -S(O)2OH), thiols (e.g., -C(S)H), sulfates, sulfonyl groups (e.g., -S(O)2-), amides (e.g., -C(O)NR2 or -N(R)C(O)R), azido groups (e.g., -N3), nitro groups (e.g., -NO2), cyano groups (e.g., -CN), isocyano groups (e.g., -NC), acyloxy groups (e.g., -OC(O)R), amino groups (e.g., -NR2, -NRH, or -NH2), carbamoyl groups (e.g., -OC(O)NR2, -OC(O)NRH, or - OC(O)NH2), sulfonamides, alkyl groups, alkenyl groups, and cyclic groups (e.g., carbocyclic groups or heterocyclic groups). In any one of the foregoing items, R is an alkyl or alkenyl group as defined herein. In some embodiments, the substituents may be further substituted with 1, 2, 3, 4, 5, or 6 substituents as defined herein. For example, a C1-6 alkyl group may be further substituted with 1, 2, 3, 4, 5, or 6 substituents as described herein.

As used herein, the term "compound" refers to all isomers and isotopes of the depicted structure "Isotopes" refer to atoms with the same atomic number, but with different mass numbers due to different neutron numbers in the nuclei. For example, isotopes of hydrogen include tritium and deuterium. In addition, a compound, salt, or complex of the present application may be prepared by a conventional method in combination with a solvent or a water molecule to form a solvate or a hydrate.

As used herein, the term "contact" refers to establishing a physical contact between two or more entities. For example, bringing a mammalian cell into contact with a nanoparticle composition means enabling the mammalian cell and nanoparticles to share a physical contact. A method of bringing a cell into contact with an external entity both in vivo and ex vivo is well-known in the field of biology. For example, a nanoparticle composition may be brought into contact with a mammalian cell in a mammal through various administration routes (e.g., intravenous, intramuscular, intradermal, and subcutaneous administration), and multiple amounts of nanoparticle composition may be involved. In addition, the nanoparticle composition may come into contact with more than one mammalian cell.

As used herein, "encapsulation efficiency" refers to the amount of a therapeutic and/or prophylactic agent that becomes a part of a nanoparticle composition, relative to the total amount of the therapeutic or prophylactic agent used in preparation of the nanoparticle composition. For example, if 97 mg of a therapeutic and/or prophylactic agent is encapsulated in a nanoparticle composition in a total of 100 mg of the therapeutic and/or prophylactic agent initially provided to the composition, the encapsulation efficiency may be 97%. As used herein, "encapsulation" may refer to complete, substantial, or partial encapsulation, closure, enclosure, or sealing.

As used herein, "expression" of a nucleic acid sequence refers to translation of mRNA into a polypeptide or a protein and/or a post-translational modification of the polypeptide or the protein.

As used herein, the term "in vitro" refers to an event that occurs in an artificial environment, for example, in a test tube or a reaction vessel, in a cell culture, and in a culture dish, rather than in an organism (e.g., an animal, a plant, or a microbe).

As used herein, the term "in vivo" refers to an event that occurs in an organism, such as an animal, a plant, or a microbe, or a cell or a tissue thereof.

As used herein, the term "ex vivo" refers to an event that occurs outside an organism (e.g., an animal, a plant, or a microbe, or a cell or a tissue thereof). An ex vivo event may occur in an environment with minimal changes to a natural (e.g., in vivo) environment.

As used herein, the term "isomer" refers to any geometric isomer, tautomer, zwitterion, stereoisomer, enantiomer, or diastereomer of a compound. A compound may include one or more chiral centers and/or double bonds, and may exist in the form of a stereoisomer, such as a double-bond isomer (i.e., a geometric E/Z isomer) or a diastereomer (e.g., an enantiomer (i.e. (+) or (-)) or a cis-trans isomer). The present application covers any and all isomers of the compound described herein. A mixture of an enantiomer and a stereoisomer of a compound, as well as a method of resolving the same into a component enantiomer or stereoisomer thereof, is well known.

As used herein, a "lipid component" refers to a component of a nanoparticle composition that includes one or more lipids. For example, a lipid component may include one or more cationic/ionizable lipids, PEGylated lipids, structural lipids, or other lipids such as phospholipids.

As used herein, an "administration method" may include intravenous, intramuscular, intradermal, subcutaneous, or other methods of delivering a composition to a subject. Any application method may be selected for targeted delivery (e.g., specific delivery) to a specific region or system of a body.

As used herein, "modified" means non-natural. For example, RNA may be a modified RNA. That is, RNA may include one or more non-naturally occurring nucleobases, nucleosides, nucleotides, or linkers. A "modified" substance may also be referred to as a "engineered" substance herein. The substance may be modified or engineered chemically, structurally, or functionally. For example, the modified nucleobase types may include one or more non-naturally occurring substitutions.

As used herein, a "nanoparticle composition" refers to a composition including one or more lipids. The particle size of a nanoparticle composition is typically on an order of micrometers or smaller, and may include a lipid bilayer. A nanoparticle composition includes a lipid nanoparticle (LNP), a liposome (e.g., a lipid vesicle), and a lipid complex. For example, a nanoparticle composition may be a liposome having a lipid bilayer and a diameter of 500 nm or less.

As used herein, "naturally occurring" refers to existing in nature without artificial help.

As used herein, a "patient" refers to a subject who may seek or require treatment, is in need of treatment, is receiving treatment, will receive treatment, or is being cared for by trained professionals for a specific disease.

As used herein, a "PEG lipid" or "PEGylated lipid" refers to a lipid containing a polyethylene glycol component.

As used herein, the term "pharmaceutically acceptable" refers to compounds, materials, compositions, and/or dosage forms that are suitable for contact with human and animal tissues within sound medical judgment without excessive toxicity, irritation, allergic reactions, or other issues or complications, commensurate with a reasonable benefit/risk ratio.

As used herein, the phrase "pharmaceutically acceptable excipient" refers to any component other than the compound described herein (e.g., a vehicle capable of suspending, complexing, or dissolving an active compound), that is substantially non-toxic and non-inflammatory to a patient. An excipient may include, for example, an anti-sticking agent, an antioxidant, a binder, a coating, a compression aid, a disintegrant, a dye (a coloring agent), an emollient, an emulsifier, a filler (a diluent), a film-forming agent or a coating, a flavoring, a fragrance, a glidant (a flow enhancer), a lubricant, a preservative, a printing ink, an adsorbent, a suspending agent or a dispersant, a sweetener, and water of hydration. An exemplary excipient includes, but is not limited to: butylated hydroxytoluene (BHT), calcium carbonate, dibasic calcium phosphate, calcium stearate, croscarmellose sodium, cross-linked polyvinylpyrrolidone, citric acid, cross-linked polyvinylpyrrolidone, cysteine, ethyl cellulose, gelatin, hydroxypropyl cellulose, hydroxypropyl methyl cellulose, lactose, magnesium stearate, maltitol, mannitol, methionine, methyl cellulose, methylparaben, microcrystalline cellulose, polyethylene glycol, polyvinylpyrrolidone, polyvinylpyrrolidone, pregelatinized starch, propylparaben, retinyl palmitate, shellac, silica, sodium carboxymethyl cellulose, sodium citrate, sodium starch glycolate, sorbitol, starch (corn), stearic acid, sucrose, talc, titanium dioxide, vitamin A, vitamin E (α-tocopherol), vitamin C, xylitol, and other substances disclosed herein.

In the present application, for convenience, a structural formula of the compound represents certain isomers, but the present application includes all isomers, such as geometric isomers, asymmetric carbon-based optically active isomers, stereoisomers, and tautomers. It should be understood that not all isomers may have the same level of activity.

The nanoparticle composition of the present application may also include one or more salts of the compound. The salts may be pharmaceutically acceptable salts. As used herein, a "pharmaceutically acceptable salt" refers to a derivative of the disclosed compound, where a parent compound is altered by converting an existing acid or basic moiety into a salt form thereof (e.g., by reacting a free base with an appropriate organic acid). Examples of pharmaceutically acceptable salts include, but are not limited to, inorganic or organic acid salts of basic residues such as amines; alkali or organic salts of acidic residues such as carboxylic acid; and the like. Representative acid addition salts include acetate, adipate, alginate, ascorbate, aspartate, benzenesulfonate, benzoate, bisulfate, borate, butyrate, camphorate, camphorsulfonate, citrate, cyclopentane propionate, digluconate, dodecyl sulfate, ethanesulfonate, fumarate, glucoheptanoate, glucoheptonate, hydrobromide, hydrochloride, hydriodate, 2-hydroxyethanesulfonate, lactate, laurate, lauryl sulfate, malate, maleate, malonate, mesylate, 2-naphthalene sulfonate, nicotinate, nitrate, oleate, oxalate, palmitate, pectate, 3-phenylpropionate, phosphate, picrate, pivalate, propionate, stearate, succinate, sulfate, tartrate, thiocyanate, tosylate, undecanoate, valerate, and the like.

Representative alkali metal or alkaline earth metal salts include sodium, lithium, potassium, calcium, magnesium, and the like, as well as non-toxic ammonium, quaternary ammonium, and amine cations, including, but not limited to ammonium, tetramethylammonium, tetraethylammonium, methylamine, dimethylamine, trimethylamine, triethylamine, ethylamine, and the like. The pharmaceutically acceptable salts of the present application include, for example, conventional non-toxic salts of a parent compound formed with non-toxic inorganic or organic acids. The pharmaceutically acceptable salts of the present application may be synthesized from a parent compound containing a basic or acidic moiety using a conventional chemical method. Typically, these salts may be prepared by reacting a free acid or base form of these compounds with a stoichiometric amount of an appropriate base or acid in water, in an organic solvent, or in a mixture of both. Typically, a non-aqueous medium such as ether, ethyl acetate, ethanol, isopropanol, or acetonitrile is preferred.

As used herein, a "phospholipid" refers to a lipid that includes a phosphate moiety and one or more carbon chains, such as unsaturated fatty acid chains. A phospholipid may include one or more multiple (e.g., double or triple) bonds (e.g., one or more unsaturated bonds). A specific phospholipid may promote fusion with a membrane. For example, a cationic phospholipid may interact with one or more negatively charged phospholipids in a membrane (e.g., a cell membrane or an intracellular membrane). Fusion of a phospholipid with a membrane allows one or more elements of a lipid containing composition to pass through the membrane, thereby allowing, for example, delivery of the one or more elements to a cell.

As used herein, a "polydispersity index" is a ratio that describes the uniformity of particle size distribution of a system. A relatively small value indicates a narrow particle size distribution.

As used herein, the term "polypeptide" or "polypeptide of interest" refers to a polymer of an amino acid residue typically linked by a peptide bond, which may be produced naturally (e.g. isolated or purified) or synthetically.

As used herein, "RNA" refers to a ribonucleic acid that may occur naturally or non-naturally. For example, RNA may include a modified and/or non-naturally occurring component, such as one or more nucleobases, nucleosides, nucleotides, or linkers. RNA may include a cap, a chain terminating nucleoside, a stem loop, a polyA sequence, and/or a polyadenylation signal. RNA may have a nucleotide sequence encoding a polypeptide of interest. For example, RNA may be a messenger RNA (mRNA). Translation of mRNA encoding a specific polypeptide, such as in vivo translation of mRNA within a mammalian cell, may produce an encoded polypeptide. RNA may be selected from a non-limiting group, including a small interfering RNA (siRNA), an asymmetric interfering RNA (aiRNA), a microRNA (miRNA), a double stranded RNA (dsRNA), a small hairpin RNA (shRNA), mRNA, and a mixture thereof.

As used herein, a "single unit dose" refers to a dose of any therapeutic agent administered in one dose/once/in a single route/at a single point of contact, i.e., a single dose of administration.

As used herein, a "divided dose" refers to dividing a single unit dose or a total daily dose into two or more doses.

As used herein, a "total daily dose" refers to an amount given or prescribed within 24 hours, which may be administered in a single unit dose.

As used herein, in the context of a nanoparticle composition, a "particle size" or an "average particle size" refers to an average diameter of the nanoparticle composition.

As used herein, the term "subject" or "patient" refers to any organism to which the composition according to the present application may be administered, for example, for experimental, diagnostic, prophylactic, and/or therapeutic purposes. Typical subjects include animals (e.g., mammals, such as mice, rats, rabbits, non-human primates, and humans) and/or plants.

The term "therapeutic agent" or "prophylactic agent" refers to any agent that has therapeutic, diagnostic, and/or prophylactic effects and/or causes a desired biological and/or pharmacological effect when administered to a subject. A therapeutic agent is also referred to as "an active agent" or "an active component". Such substances include, but are not limited to, cytotoxins, radioactive ions, chemotherapeutic agents, small molecule drugs, proteins, and nucleic acids.

As used herein, the term "therapeutically effective amount" refers to a sufficient amount of an agent to be delivered (e.g., nucleic acids, a drug, a composition, a therapeutic agent, a diagnostic agent, and a prophylactic agent) when given to a subject who is suffering from or susceptible to an infection, disease, disorder, and/or condition to treat, ameliorate symptoms, diagnose, prevent, and/or delay onset of the infection, disease, disorder, and/or condition.

As used herein, "transfection" refers to the introduction of a species (e.g., RNA) into a cell. Transfection may be conducted, for example, in vitro, ex vivo, or in vivo.

As used herein, the term "treatment" refers to partial or complete remission, relief, improvement, alleviation, delay of onset, inhibition of progression, reduction of severity, and/or reduction of the incidence of one or more symptoms or features of a specific infection, disease, disorder, and/or condition. For example, "treatment" of cancer may refer to inhibiting the survival, growth, and/or spread of tumors. To reduce risk, treatment may be given to a subject who does not exhibit a disease, disorder, and/or condition, and/or to a subject who only exhibits early signs of a disease, disorder, and/or condition. Pathological development related to a disease, disorder, and/or condition.

The present application discloses a compound of formula (I), or a salt or an isomer thereof, 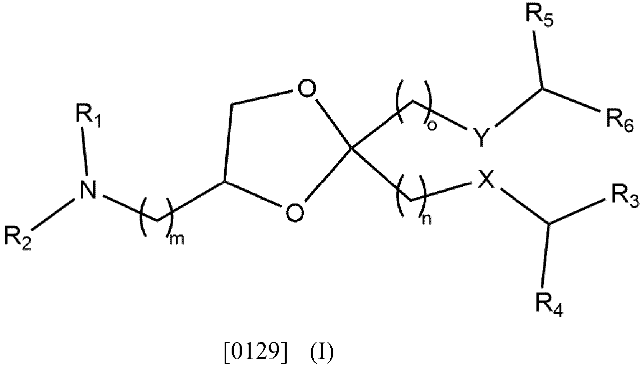
where R1 and R2 are independently C1-C6 alkyl groups; R3 and R4 are independently C1-C12 alkyl groups; R5 and R6 are independently selected from hydrogen or C1-C12 alkyl groups;
X is selected from (C=O)O or O(C=O), and Y and Z are independently selected from (C=O)O or O(C=O); and
m, n, and o are independently selected from 1, 2, 3, 4, 5, 6, 7, 8, 9, and 10.

In a preferred embodiment, R1 is selected from C1-C6 alkyl groups, for example, R1 is selected from C1-C5 alkyl groups, C1-C4 alkyl groups, or C1-C3 alkyl groups, or for example, R1 is a C1 alkyl group, a C2 alkyl group, a C3 alkyl group, a C4 alkyl group, a C5 alkyl group, or a C6 alkyl group.

In a preferred embodiment, R1 is selected from C1-C6 linear alkyl groups, for example, R1 is selected from C1-C5 linear alkyl groups, C1-C4 linear alkyl groups, or C1-C3 linear alkyl groups, or for example, R1 is a C1 alkyl group, a C2 alkyl group, a C3 linear alkyl group, a C4 linear alkyl group, a C5 linear alkyl group, or a C6 linear alkyl group. In a further preferred embodiment, R1 is a methyl group or an ethyl group. In a further preferred embodiment, R1 is a methyl group.

In a preferred embodiment, R2 is selected from C1-C6 alkyl groups, for example, R2 is selected from C1-C5 alkyl groups, C1-C4 alkyl groups, or C1-C3 alkyl groups, or for example, R2 is a C1 alkyl group, a C2 alkyl group, a C3 alkyl group, a C4 alkyl group, a C5 alkyl group, or a C6 alkyl group.

In a preferred embodiment, R2 is selected from C1-C6 linear alkyl groups, for example, R2 is selected from C1-C5 linear alkyl groups, C1-C4 linear alkyl groups, or C1-C3 linear alkyl groups, or for example, R2 is a C1 alkyl group, a C2 alkyl group, a C3 linear alkyl group, a C4 linear alkyl group, a C5 linear alkyl group, or a C6 linear alkyl group. In a further preferred embodiment, R2 is a methyl group or an ethyl group. In a further preferred embodiment, R2 is a methyl group.

In a preferred embodiment, R3 is selected from C1-C12 alkyl groups, for example, R3 is selected from C2-C12 alkyl groups, C3-C12 alkyl groups, C4-C12 alkyl groups, C5-C12 alkyl groups, C6-C12 alkyl groups, C6-C8 alkyl groups, C7-C12 alkyl groups, C8-C12 alkyl groups, C9-C12 alkyl groups, C10-C12 alkyl groups, or C11-C12 alkyl groups, or for example, R3 is a C1 alkyl group, a C2 alkyl group, a C3 alkyl group, a C4 alkyl group, a C5 alkyl group, a C6 alkyl group, a C7 alkyl group, a C8 alkyl group, a C9 alkyl group, a C10 alkyl group, a C11 alkyl group, or a C12 alkyl group. In a further preferred embodiment, R3 is a C8 alkyl group or a C6 alkyl group.

In a preferred embodiment, R3 is selected from C1-C12 linear alkyl groups, for example, R3 is selected from C2-C12 linear alkyl groups, C3-C12 linear alkyl groups, C4-C12 linear alkyl groups, C5-C12 linear alkyl groups, C6-C12 linear alkyl groups, C6-C8 linear alkyl groups, C7-C12 linear alkyl groups, C8-C12 linear alkyl groups, C9-C12 linear alkyl groups, C10-C12 linear alkyl groups, or C11-C12 linear alkyl groups, or for example, R3 is a C1 linear alkyl group, a C2 linear alkyl group, a C3 linear alkyl group, a C4 linear alkyl group, a C5 linear alkyl group, a C6 linear alkyl group, a C7 linear alkyl group, a C8 linear alkyl group, a C9 linear alkyl group, a C10 linear alkyl group, a C11 linear alkyl group, or a C12 linear alkyl group. In a further preferred embodiment, R3 is a C8 linear alkyl group or a C6 linear alkyl group.

In a preferred embodiment, R4 is selected from C1-C12 alkyl groups, for example, R4 is selected from C2-C12 alkyl groups, C3-C12 alkyl groups, C4-C12 alkyl groups, C5-C12 alkyl groups, C6-C12 alkyl groups, C6-C8 alkyl groups, C7-C12 alkyl groups, C8-C12 alkyl groups, C9-C12 alkyl groups, C10-C12 alkyl groups, or C11-C12 alkyl groups, or for example, R3 is a C1 alkyl group, a C2 alkyl group, a C3 alkyl group, a C4 alkyl group, a C5 alkyl group, a C6 alkyl group, a C7 alkyl group, a C8 alkyl group, a C9 alkyl group, a C10 alkyl group, a C11 alkyl group, or a C12 alkyl group. In a further preferred embodiment, R4 is a C8 alkyl group or a C6 alkyl group.

In a preferred embodiment, R4 is selected from C1-C12 linear alkyl groups, for example, R4 is selected from C2-C12 linear alkyl groups, C3-C12 linear alkyl groups, C4-C12 linear alkyl groups, C5-C12 linear alkyl groups, C6-C12 linear alkyl groups, C6-C8 linear alkyl groups, C7-C12 linear alkyl groups, C8-C12 linear alkyl groups, C9-C12 linear alkyl groups, C10-C12 linear alkyl groups, or C11-C12 linear alkyl groups, or for example, R4 is a C1 linear alkyl group, a C2 linear alkyl group, a C3 linear alkyl group, a C4 linear alkyl group, a C5 linear alkyl group, a C6 linear alkyl group, a C7 linear alkyl group, a C8 linear alkyl group, a C9 linear alkyl group, a C10 linear alkyl group, a C11 linear alkyl group, or a C12 linear alkyl group. In a further preferred embodiment, R4 is a C8 linear alkyl group or a C6 linear alkyl group.

In a preferred embodiment, R5 is H, and R6 is selected from C1-C12 alkyl groups, for example, R6 is selected from C2-C12 alkyl groups, C3-C12 alkyl groups, C4-C12 alkyl groups, C5-C12 alkyl groups, C6-C12 alkyl groups, C6-C8 alkyl groups, C7-C12 alkyl groups, C8-C12 alkyl groups, C9-C12 alkyl groups, C10-C12 alkyl groups, or C11-C12 alkyl groups, or for example, R6 is a C1 alkyl group, a C2 alkyl group, a C3 alkyl group, a C4 alkyl group, a C5 alkyl group, a C6 alkyl group, a C7 alkyl group, a C8 alkyl group, a C9 alkyl group, a C10 alkyl group, a C11 alkyl group, or a C12 alkyl group. In a further preferred embodiment, R5 is H, and R6 is a C9-C11 alkyl group. In a further preferred embodiment, R5 is H, and R6 is a C9 alkyl group, a C10 alkyl group, or a C11 alkyl group.

In a preferred embodiment, R5 is H, and R6 is selected from C1-C12 linear alkyl groups, for example, R6 is selected from C2-C12 linear alkyl groups, C3-C12 linear alkyl groups, C4-C12 linear alkyl groups, C5-C12 linear alkyl groups, C6-C12 linear alkyl groups, C6-C8 linear alkyl groups, C7-C12 linear alkyl groups, C8-C12 linear alkyl groups, C9-C12 linear alkyl groups, C10-C12 linear alkyl groups, or C11-C12 linear alkyl groups, or for example, R6 is a C1 linear alkyl group, a C2 linear alkyl group, a C3 linear alkyl group, a C4 linear alkyl group, a C5 linear alkyl group, a C6 linear alkyl group, a C7 linear alkyl group, a C8 linear alkyl group, a C9 linear alkyl group, a C10 linear alkyl group, a C11 linear alkyl group, or a C12 linear alkyl group. In a further preferred embodiment, R5 is H, and R6 is a C9-C11 linear alkyl group. In a still further preferred embodiment, R5 is H, and R6 is a C9 linear alkyl group, a C10 linear alkyl group, or a C11 linear alkyl group.

In a preferred embodiment, R5 and R6 are independently selected from C1-C12 alkyl groups, for example, from C2-C12 alkyl groups, C3-C12 alkyl groups, C4-C12 alkyl groups, C5-C12 alkyl groups, C6-C12 alkyl groups, C6-C8 alkyl groups, C7-C12 alkyl groups, C8-C12 alkyl groups, C9-C12 alkyl groups, C10-C12 alkyl groups, or C11-C12 alkyl groups, or for example, R5 and R6 are independently a C1 alkyl group, a C2 alkyl group, a C3 alkyl group, a C4 alkyl group, a C5 alkyl group, a C6 alkyl group, a C7 alkyl group, a C8 alkyl group, a C9 alkyl group, a C10 alkyl group, a C11 alkyl group, or a C12 alkyl group. In a further preferred embodiment, R5 is a C6 alkyl group and R6 is a C8 alkyl group, or R6 is a C6 alkyl group and R5 is a C8 alkyl group.

In a preferred embodiment, R5 and R6 are independently selected from C1-C12 linear alkyl groups, for example, from C2-C12 linear alkyl groups, C3-C12 linear alkyl groups, C4-C12 linear alkyl groups, C5-C12 linear alkyl groups, C6-C12 linear alkyl groups, C6-C8 linear alkyl groups, C7-C12 linear alkyl groups, C8-C12 linear alkyl groups, C9-C12 linear alkyl groups, C10-C12 linear alkyl groups, or C11-C12 linear alkyl groups, or for example, R5 and R6 are independently a C1 linear alkyl group, a C2 linear alkyl group, a C3 linear alkyl group, a C4 linear alkyl group, a C5 linear alkyl group, a C6 linear alkyl group, a C7 linear alkyl group, a C8 linear alkyl group, a C9 linear alkyl group, a C10 linear alkyl group, a C11 linear alkyl group, or a C12 linear alkyl group. In a further preferred embodiment, R5 is a C6 linear alkyl group and R6 is a C8 linear alkyl group, or R6 is a C6 linear alkyl group and R5 is a C8 linear alkyl group.

In a preferred embodiment, m is 3, 4, 5, 6, or 7. In a further preferred embodiment, m is 4, 5, or 6.

In a preferred embodiment, n is selected from 4, 5, 6, 7, 8, and 9. In a further preferred embodiment, n is 5, 6, 7, or 8.

In a preferred embodiment, o is 4, 5, 6, 7, 8, or 9. In a further preferred embodiment, o is 5, 6, 7, or 8.

In a preferred embodiment, m is 4.

In a preferred embodiment, n is 6.

In a preferred embodiment, o is 6 or 8.

In a preferred embodiment, X is (C=O)O and Y is O(C=O), or X is (C=O)O and Y is (C=O)O, or X is O(C=O) and Y is O(C=O), or X is O(C=O) and Y is (C=O)O. In a further preferred embodiment, X is O(C=O) and Y is O(C=O).

The present application further provides a nanoparticle composition including a lipid component, where the lipid component includes the compound of formula (I), or the salt or the isomer thereof provided in the present application.

In some embodiments, an average particle size of the nanoparticle composition is 60-130 nm.

The nanoparticle composition may include, for example, a lipid nanoparticle (LNP), a liposome, a lipid vesicle, and a lipid complex.

The nanoparticle composition of the present application includes a lipid component, and the lipid component includes at least one of the compound of formula (I), or the salt or the isomer thereof. For example, the lipid component of the nanoparticle composition may include one or more of the compounds of the present invention. The nanoparticle composition may also include various other components. For example, in addition to the compound of formula (I), the lipid component of the nanoparticle composition may also include one or more other lipids.

The lipid component of the nanoparticle composition may include one or more PEG lipids or PEG-modified lipids. Such substances may alternatively be referred to as PEGylated lipids. PEG lipids are PEG-modified lipids. PEG lipids may be selected from a non-limiting group consisting of PEG-modified phosphatidylethanolamine, PEG-modified phosphatidic acid, PEG-modified ceramide, PEG-modified dialkylamine, PEG-modified diacylglycerol, PEG-modified dialkylglycerol, and a mixture thereof. For example, PEG lipids may be PEG-c-DOMG, PEG-DMG, PEG-DLPE, PEG-DMPE, PEG-DPPC, or PEG-DSPE lipids, preferably, DMG-PEG2000.

The lipid component of the nanoparticle composition may include one or more structural lipids. The structural lipids may be selected from, but are not limited to, cholesterol, coprosterol, sitosterol, ergosterol, stigmasterol, and a mixture thereof. In some embodiments, the structural lipid is cholesterol. In some embodiments, the structural lipids include cholesterol and corticosteroids (e.g., prednisolone, dexamethasone, prednisone, and hydrocortisone), or a combination thereof.

The lipid component of the nanoparticle composition may include one or more phospholipids, and the phospholipids used in the nanoparticle composition and method may be selected from:
dilauroyl phosphatidylcholine (DLPC),
dimyristoyl phosphatidylcholine (DMPC),
dioleoyl phosphatidylcholine (DOPC),
dipalmitoyl phosphatidylcholine (DPPC),
distearoyl phosphatidylcholine (DSPC),
dioleoyl phosphatidylcholine (DUPC),
palmitoyl oleoyl phosphatidylcholine (POPC),
1,2-di-O-octadecyl-sn-glycero-3-phosphocholine (18:0 Diether PC), 1- oleoyl-2-cholesterylhemisuccinoyl-sn-glycero-3-phosphocholine (OChemsPC),
1-hexadecyl-sn-glycero-3-phosphocholine (C16 Lyso PC),
1,2-divinyl-sn-glycero-3-phosphocholine,
1,2-diarylacyl-sn-glycero-3-phosphocholine,
1,2-dioleoyl-SN-glycero-3-phosphoethanolamine (DOPE),
1,2-distearoyl-sn-glycero-3-phosphoethanolamine,
1,2-divinylol-sn-glycero-3-phosphoethanolamine,
1,2-divinyl-sn-glycero-3-phosphoethanolamine,
1,2-diaryl-sn-glycero-3-phosphoethanolamine,
1,2-dithiohexaenoyl-sn-glycero-3-phosphoethanolamine, and
1,2- diolyl -sn-glycero-3-phosphatidyl-(1-glycerol) sodium salt (DOPG) or sphingomyelin.

In some embodiments, the nanoparticle composition includes DSPC. In some embodiments, the nanoparticle composition includes DOPE. In some embodiments, the nanoparticle composition includes DSPC and DOPE.

In some embodiments, the LNP includes an ionizable lipid, a phospholipid, cholesterol, and a PEG lipid, where the content of the ionizable lipid is 35-65 mol%, the combined content of the phospholipid and the cholesterol is 35-65 mol%, and the content of the PEG lipid is 0.5-5 mol%.

The nanoparticle composition may include one or more therapeutic agents and/or prophylactic agents, where the therapeutic agents and/or the prophylactic agents are selected from a vaccine, or a compound and a nucleic acid enabling triggering of an immune response, and preferably, the nucleic acid is RNA selected from one or more of siRNA, aiRNA, miRNA, dsRNA, shRNA, or mRNA.

The present application provides a method for delivering a therapeutic agent and/or a prophylactic agent to a mammalian cell or organ, producing a polypeptide of interest in the mammalian cell, and treating a disease or disorder in a mammal in need thereof. The method includes administering to a mammal and/or bringing a mammalian cell into contact with a composition including therapeutic and/or prophylactic nanoparticles.

In some embodiments, the therapeutic agent and/or the prophylactic agent is mRNA. The mRNA may encode any polypeptide of interest, including any naturally or non-naturally occurring or otherwise modified polypeptide. The polypeptide encoded by the mRNA may have any size and may have any secondary structure or activity. In some embodiments, the polypeptide encoded by the mRNA may have a therapeutic effect when expressed in a cell.

The lipid component of the nanoparticle composition may include, for example, the compound of formula (I), a phospholipid (e.g., an unsaturated lipid, such as DOPE or DSPC), a PEG lipid, and a structural lipid.

The nanoparticle composition may be characterized by various methods. For example, a microscope (e.g., a transmission electron microscope or a scanning electron microscope) may be used to examine the morphology and size distribution of the nanoparticle composition. The ζ potential may be measured by dynamic light scattering or potentiometry (e.g., potentiometric titration). The particle size may also be determined by dynamic light scattering.

The average particle size of the nanoparticle composition is 60-130 nm.

The nanoparticle composition may be relatively uniform. A polydispersity index may be used to represent the uniformity of the nanoparticle composition, for example, particle size distribution of the nanoparticle composition. A small polydispersity index typically indicates a narrow particle size distribution.

The present application further provides a method for specifically delivering a therapeutic agent and/or a prophylactic agent to an organ of a mammal, including administering the nanoparticle composition according to any one of the foregoing items to the mammal, the administering including bringing the organ of the mammal into contact with the nanoparticle composition to deliver the therapeutic agent and/or the prophylactic agent to the organ. The therapeutic agent and/or the prophylactic agent, for example, proteins, a cytotoxic agent, radioactive ions, a chemotherapeutic agent, or nucleic acids (e.g., RNA, such as mRNA), may be delivered to the cell or organ. In a case that the therapeutic agent and/or the prophylactic agent is mRNA, when a cell comes into contact with the nanoparticle composition, a translatable mRNA may be translated in the cell to produce a polypeptide of interest. However, substantially untranslatable mRNA may also be delivered to a cell. The substantially untranslatable mRNA may be used as vaccines and/or may sequester translational machinery of the cell to reduce expression of other species in the cell.

In some embodiments, the nanoparticle composition may target a specific type or class of cells (e.g., cells of a specific organ or system). For example, a composition including target therapeutic and/or prophylactic nanoparticles may be specifically delivered to the liver, kidney, spleen, gastrointestinal tract, femur, or lung of a mammal. Specific delivery to a specific class of cells, organs, systems, or groups means that a higher proportion of the nanoparticle composition including the therapeutic and/or prophylactic agent is delivered to a target site (e.g., a tissue) compared to other sites, for example, when the nanoparticle composition is administered to a mammal. In some embodiments, the target tissue is selected from liver, kidney, lung, spleen, femur, gastrointestinal tract, ocular tissue (e.g., via intraocular, subretinal, or intravitreal injection), vascular endothelium in a blood vessel (e.g., intracoronary or intrafemoral) or kidney, and tumor tissue (e.g., via intratumoral injection).

### Examples

### Example 1 Synthesis of Compound 1

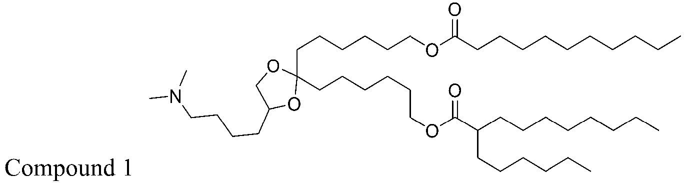

A synthesis route is shown below.

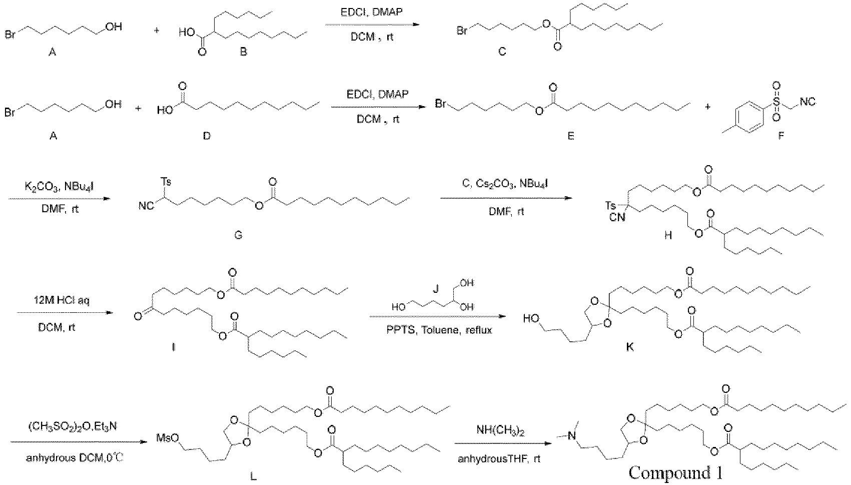

Specifically, Compound A (11.2 g, 53.2 mmol), EDCI (12.2 g, 63.84 mmol), and DMAP (2.6 g, 22.0 mmol) were added to a solution of Compound B (15.0 g, 58.5 mmol) in dichloromethane (50 ml). The mixture was stirred at room temperature for 7 h, and the reaction was monitored by TLC. After completion of the reaction, the reaction solution was diluted with an equal volume of saturated sodium bicarbonate solution and separated. The organic phase was dried over anhydrous magnesium sulfate and filtered. The filtrate was concentrated and subjected to silica gel column chromatography (petroleum ether:ethyl acetate=25:1) to obtain Compound C (20.5 g, 86.35%).

Compound A (15.0 g, 82.84 mmol), EDCI (20.0 g, 107.69 mmol), and DMAP (4.0 g, 33.13 mmol) were added to a solution of Compound D (18.5 g, 99.30 mmol) in dichloromethane (100 ml). The mixture was stirred at room temperature for 7 h, and the reaction was monitored by TLC. After completion of the reaction, the reaction solution was diluted with an equal volume of saturated sodium bicarbonate solution and separated. The organic phase was dried over anhydrous magnesium sulfate and filtered. The filtrate was concentrated and subjected to silica gel column chromatography (petroleum ether:ethyl acetate=30:1) to obtain Compound E (18.9 g, 65.63%).

Compound F (5.6 g, 28.72 mmol), NBu4I (5.2 g, 14.36 mmol), and K₂CO₃ (3.9 g, 28.72 mmol) were added to a solution of Compound E (5.0 g, 14.36 mmol) in DMF (30 ml). The mixture was stirred at room temperature for 12 h, and the reaction was monitored by TLC. After completion of the reaction, the reaction solution was diluted with an equal volume of water, extracted with ethyl acetate (10 ml×3), concentrated, and subjected to silica gel column chromatography (petroleum ether:ethyl acetate=20:1) to obtain Compound G (3.4 g, yield 51.12%).

Compound C (7.5 g, 17.81 mmol), NBu4I (4.4 g, 11.91 mmol), and Cs2CO3 (7.7 g, 23.70 mmol) were added to a solution of Compound G (5.5 g, 11.91 mmol) in DMF (20 ml). The mixture was stirred at room temperature for 15 h, and the reaction was monitored by TLC. After completion of the reaction, the reaction solution was diluted with an equal volume of water, extracted with ethyl acetate (20 ml×3), concentrated, and subjected to silica gel column chromatography (petroleum ether:ethyl acetate=20:1) to obtain Compound H (5.9 g, yield 62.11%).

Concentrated hydrochloric acid (20 ml) was added to a solution of Compound H (5.9 g, 7.36 mmol) in dichloromethane (50 ml). The mixture was stirred at room temperature for 3 h, and the reaction was monitored by TLC. After completion of the reaction, the reaction solution was separated, and the organic layer was washed with a saturated sodium bicarbonate solution, dried over anhydrous magnesium sulfate, concentrated, and subjected to silica gel column chromatography (petroleum ether:ethyl acetate=30:1) to obtain Compound I (3.8 g, 81.19%).

Compound J (0.6 g, 5.2 mmol) and pyridinium p-toluenesulfonate (0.4 g, 1.72 mmol) were added to a solution of Compound I (1.1 g, 1.72 mmol) in toluene (25 ml) and heated under reflux using a Dean-Stark apparatus for 20 h, and the reaction was monitored by TLC. After completion of the reaction, the reaction solution was cooled to room temperature, diluted with an equal volume of water, extracted with ethyl acetate (10 ml×3), concentrated, and subjected to silica gel column chromatography (petroleum ether:ethyl acetate=5:1) to obtain Compound K (0.7 g, yield 53.85%).

Methanesulfonic anhydride (0.32 g, 1.85 mmol) and anhydrous triethylamine (0.28 ml) were added to a solution of Compound K (0.7 g, 0.93 mmol) in ultra-dry dichloromethane (10 ml) at 0°C. The mixture was stirred for 12 h, and the reaction was monitored by TLC. After completion of the reaction, the reaction solution was allowed to warm to room temperature, diluted with an equal volume of water and separated. The organic layer was dried over anhydrous magnesium sulfate and filtered. The filtrate was concentrated and subjected to silica gel column chromatography (petroleum ether:ethyl acetate=5:1) to obtain Compound L (0.84 g, yield 97%).

A solution (30 ml) of dimethylamine (2 M in THF) was added to Compound L (0.84 g, 1.01 mmol). The mixture was sealed and stirred at room temperature for 6 days, and the reaction was monitored by TLC. After completion of the reaction, the reaction solution was concentrated and subjected to silica gel column chromatography (dichloromethane: methanol=30:1) to obtain oily Compound 1 (249 mg, yield 31.58%).

¹H NMR (600 MHz, CDCl₃) δ 4.08 - 3.96 (m, 6H), 3.42 (d, *J* = 5.8 Hz, 1H), 2.37 (t, 2H), 2.31 (s, 6H), 2.29 - 2.24 (m, 3H), 1.66 - 1.52 (m, 15H), 1.49 - 1.35 (m, 5H), 1.34 - 1.17 (m, 46H), 0.86 (t, *J* = 7.0 Hz, 9H). MS-ESI (m/z): 781.0(M+H)⁺.

### Example 2 Synthesis of Compound 2

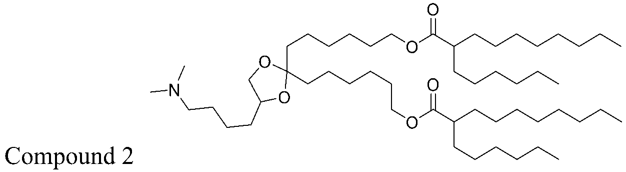

A synthesis route is shown below.

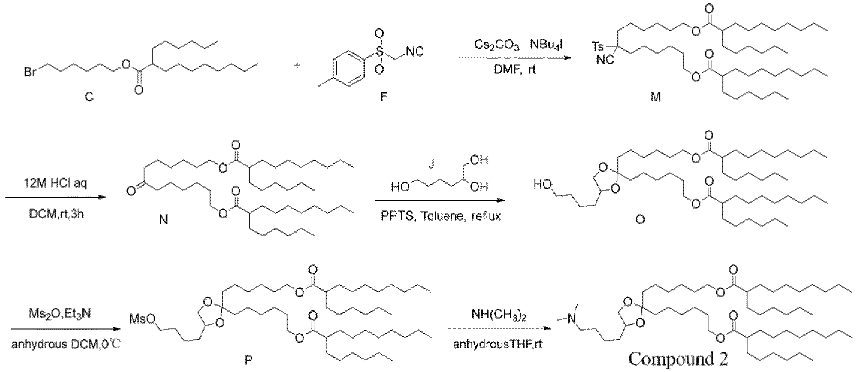

Compound F (1.4 g, 7.19 mmol), NBu4I (3.0 g, 7.19 mmol), and Cs₂CO₃ (7.1 g, 21.58 mmol) were added to a solution of Compound C (10.0 g, 21.58 mmol) in DMF (50 ml). The mixture was stirred at room temperature for 12 h, and the reaction was monitored by TLC. After completion of the reaction, the reaction solution was diluted with an equal volume of water, extracted with ethyl acetate (50 ml×3), concentrated, and subjected to silica gel column chromatography (petroleum ether:ethyl acetate=20:1) to obtain Compound M (5.0 g, yield 80.64%).

Concentrated hydrochloric acid (20 ml) was added to a solution of Compound M (5.0 g, 5.73 mmol) in dichloromethane (50 ml). The mixture was stirred at room temperature for 3 h, and the reaction was monitored by TLC. After completion of the reaction, the reaction solution was separated, and the organic phase was washed with a saturated sodium bicarbonate solution, dried over anhydrous magnesium sulfate, concentrated, and subjected to silica gel column chromatography (petroleum ether:ethyl acetate=30:1) to obtain Compound N (3.75 g, 92.59%).

Compound J (0.6 g, 4.71 mmol) and pyridinium p-toluenesulfonate (0.6 g, 2.30 mmol) were added to a solution of Compound N (1.1 g, 1.56 mmol) in toluene (25 ml) and heated under reflux using a Dean-Stark apparatus for 20 h, and the reaction was monitored by TLC. After completion of the reaction, the reaction solution was cooled to room temperature, diluted with an equal volume of water, extracted with ethyl acetate (10 ml×3), concentrated, and subjected to silica gel column chromatography (petroleum ether:ethyl acetate=5:1) to obtain Compound O (1.3 g, yield 91.87%).

Methanesulfonic anhydride (0.38 g, 2.18 mmol) and anhydrous triethylamine (0.28 ml) were added to a solution of Compound O (0.9 g, 1.09 mmol) in ultra-dry dichloromethane (10 ml) at 0°C. The mixture was stirred for 12 h, and the reaction was monitored by TLC. After completion of the reaction, the reaction solution was allowed to warm to room temperature, diluted with an equal volume of water and separated. The organic layer was dried over anhydrous magnesium sulfate and filtered. The filtrate was concentrated and subjected to silica gel column chromatography (petroleum ether:ethyl acetate=5:1) to obtain Compound P (0.93 g, yield 95%).

A solution (30 ml) of dimethylamine (2 M in THF) was added to Compound P (0.93 g, 1.04 mmol). The mixture was sealed and stirred at room temperature for 6 days, and the reaction was monitored by TLC. After completion of the reaction, the reaction solution was concentrated and subjected to silica gel column chromatography (dichloromethane: methanol=30:1) to obtain oily Compound 2 (300 mg, yield 34.13%).

¹H NMR (600 MHz, Chloroform-*d*) δ 4.08 - 3.94 (m, 6H), 3.42 (t, *J* = 6.2 Hz, 1H), 2.40 - 2.34 (m, 2H), 2.32 - 2.24 (m, 8H), 1.63 - 1.53 (m, 14H), 1.44 - 1.38 (m, 5H), 1.36 - 1.30 (m, 11H), 1.24 (s, 44H), 0.86 (t, *J* = 6.3 Hz, 12H).MS-ESI (m/z): 851.0(M+H)⁺.

### Example 3 Synthesis of Compound 3

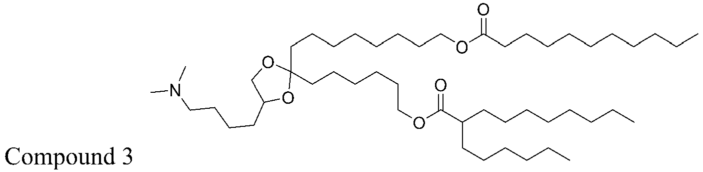

A preparation method is the same as that of Compound 1, and Compound A was substituted with 8-bromo-1-octanol as a starting material to react with Compound D to obtain oily Compound 3.

¹H NMR (600 MHz, CDCl₃) δ 4.09 - 3.99 (m, 6H), 3.45 - 3.37 (m, 1H), 2.33 (t, *J* = 7.6 Hz, 2H), 2.31 - 2.24 (m, 9H), 1.67 - 1.47 (m, 16H), 1.44 - 1.38 (m, 3H), 1.37 - 1.20 (m, 51H), 0.87 (t, *J* = 7.0 Hz, 9H). MS-ESI (m/z): 809.0(M+H)⁺.

### Example 4 Synthesis of Compound 4

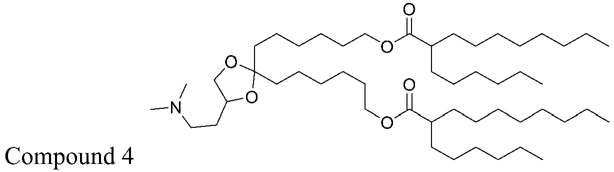

A preparation method is the same as that of Compound 2, and Compound J was substituted with 1,2,4-butanetriol as a starting material to obtain oily Compound 4.

¹H NMR (600 MHz, Chloroform-d) δ 4.11 - 4.02 (m, 6H), 3.47 (t, *J =* 7.6 Hz, 1H), 2.47 - 2.41 (m, 1H), 2.37 - 2.33 (m, 1H), 2.32 - 2.24 (m, 8H), 1.84 - 1.78 (m, 1H), 1.72 - 1.65 (m, 1H), 1.65 - 1.53 (m, 12H), 1.46 - 1.38 (m, 4H), 1.37 - 1.31 (m, 10H), 1.30 - 1.21 (m, 42H), 0.87 (t, *J* = 7.6 Hz, 12H).

### Example 5 Synthesis of Compound 5

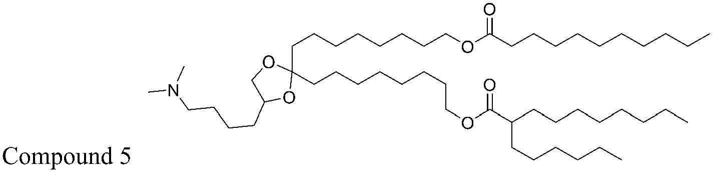

A preparation method is the same as that of Compound 1, and Compound A was substituted with 8-bromo-1-octanol as a starting material to obtain oily Compound 5.

¹H NMR (500 MHz, CDCl₃) δ 4.09 - 3.99 (m, 6H), 3.46 - 3.37 (m, 1H), 2.41 (t,*J =* 7.7 Hz, 2H), 2.34 (s, 6H), 2.28 (t, *J* = 7.6 Hz, 3H), 1.68 - 1.37 (m, 19H), 1.26 (d, *J =* 18.7 Hz, 55H), 0.87 (t, *J =* 5.9 Hz, 9H). MS-ESI (m/z): 836.6(M+H)⁺.

### Example 6 Synthesis of control Compound NTX-C16 (WO2023184038A1)

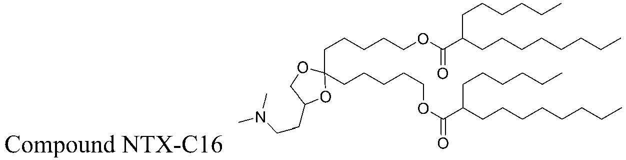

A preparation method is the same as that of Compound 2, and Compound A was substituted with 5-bromo-1-pentanol as a starting material to obtain oily Compound NTX-C16.

¹H NMR (400 MHz, CDCl₃) δ 4.18 - 4.05 (m, 6H), 3.58 - 3.45 (m, 1H), 2.51 - 2.32 (m, 4H), 2.30 (s, 6H), 1.85 - 1.59(m, 14H), 1.47 - 1.39(m, 8H), 1.37 - 1.24(m, 44H), 0.91 (t, *J* = 6.8 Hz, 12H). MS-ESI (m/z): 794.8(M+H)⁺.

### Test Examples

### Test Example 1 Encapsulation by lipid nanoparticles (LNPs)

An mRNA stock solution was dispersed in a 20 mM acetic acid solution (pH 5.3) to a final concentration of 200 µg/mL (aqueous phase). The example compound, cholesterol, DSPC, and DMG-PEG2000 were mixed in a molar ratio of 50:38.5:10:1.5 to form a mixed lipid (oil phase). Flow rates of the aqueous phase and the oil phase were controlled to mix mRNA with the lipid mixture via T-junction mixing, to obtain LNP encapsulated mRNA. The encapsulated LNPs were diluted with a buffer solution and then concentrated by ultrafiltration. The diluent was replaced. Finally, the LNPs were concentrated to an mRNA concentration of 100 µg/mL, and the pH of the LNPs was adjusted to about 7.0-8.5. Finally, the total mRNA content and free mRNA content in the LNPs were measured by using a RiboGreen assay kit and 10% OTG as a demulsifier, and the encapsulation efficiency of the LNPs was calculated. The final LNP product was diluted with a diluent, added (1 ml) to a particle size cell, and placed on a Malvern Zetasizer instrument to measure the particle size of the LNPs. The results are shown in Table 1.

The particle size, PDI, and encapsulation efficiency are important quality attributes of lipid nanoparticles. From the table below, the tested compounds have satisfactory encapsulation efficiencies, suitable particle size for mRNA delivery, and narrow PDI.

**Table 1 Characterization data of LNPs of compounds in examples**

| Compound No. | Encapsulation efficiency/% | Particle size/nm | PDI |
|---|---|---|---|
| Compound 1 | 97.16 | 116.9 | 0.04995 |
| Compound 2 | 98.71 | 79.91 | 0.0152 |
| Compound 3 | 91.45 | 111.7 | 0.07126 |
| Compound 4 | 95.88 | 69.74 | 0.09896 |
| Compound 5 | 97.00 | 136.8 | 0.1833 |
| Compound NTX-C16 | 95.50 | 64.56 | 0.1372 |
| Compound MC3 | 92.17 | 74.36 | 0.1415 |

### Test Example 2 Evaluation of Delivery Efficiency

1. Luciferase mRNA was encapsulated with LNP formulations of Compounds 1 to 3, NTX-C16, and MC3. A method for preparing the LNP formulations and a method for encapsulating the mRNA are as described in Test Example 1.
2. The encapsulated LNP formulations were intramuscularly injected into BALB/c mice (injection dose: 0.25 mg/kg muscle), with 5 mice in each group.
3. The luciferase fluorescence expression intensity of each mouse was measured at 24 h.
4. Measurement of fluorescence expression intensity: D-Luciferin sodium salt (dose: 150 mg/kg) was intraperitoneally injected into each mouse 10 min before measurement. Then the mouse was anesthetized with isoflurane and placed in an IVIS instrument for measurement using bioluminescence.
5. The delivery efficiency of each compound was evaluated based on the fluorescence intensity results

A luciferase assay is a main method for detecting mRNA expression in vivo by means of fluorescence intensity. According to the results in FIG. 1, 24 h after intramuscular injection, the expression of Compounds 1 and 3 with a single-double type structure at the tail was significantly higher than that of Compound 2 with a double-double type structure at the tail and that of the Compound NTX-C16 in the prior art.

The foregoing results show that Compounds 1 and 3 have excellent mRNA delivery performance and are superior to the compounds in the prior art.

Although the embodiments of the present application are described above, the present application is not limited to the foregoing specific embodiments and application fields, and the foregoing specific embodiments are merely exemplary and instructive rather than limiting. Under the teaching of this specification, a person of ordinary skill in the art may further make various forms without departing from the protection scope of the claims of the present application, and all the forms fall within the protection scope of the present application.

## Claims

1. A compound of formula (I), or a pharmaceutically acceptable salt or a stereoisomer thereof: 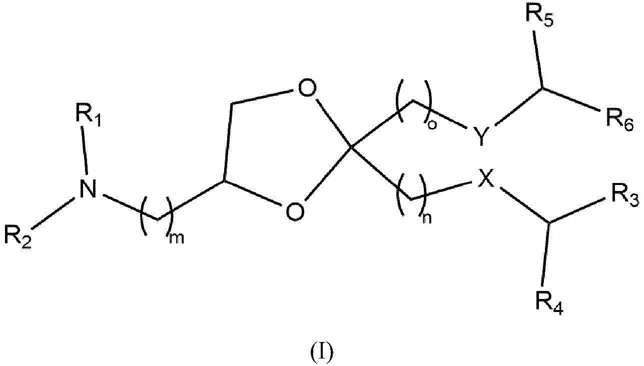
wherein R₁ and R₂ are independently C1-C6 alkyl groups;
R₃ and R₄ are each independently a C5, C6, C7, C8, or C9 linear alkyl group;
R₅ is hydrogen; R₆ is a C8, C9, C10, or C11 linear alkyl group;
X and Y are independently selected from (C=O)O or O(C=O);
m is 4;
o is independently selected from 5, 6, 7, or 8; and
n is independently selected from 5, 6, 7, or 8.

2. The compound, or the pharmaceutically acceptable salt or the stereoisomer thereof according to any one of the preceding claims, wherein R₁ and R₂ are each independently a C1-C3 alkyl group, preferably a methyl group.

3. The compound, or the pharmaceutically acceptable salt or the stereoisomer thereof according to any one of the preceding claims, wherein R₆ is a C9 linear alkyl group.

4. The compound, or the pharmaceutically acceptable salt or the stereoisomer thereof according to any one of the preceding claims, wherein R₅ and R₆ are each independently a C6 or C8 linear alkyl group.

5. The compound, or the pharmaceutically acceptable salt or the stereoisomer thereof according to any one of the preceding claims, wherein X is (C=O)O and Y is O(C=O), or X is (C=O)O and Y is (C=O)O, or X is O(C=O) and Y is O(C=O), or X is O(C=O) and Y is (C=O)O, preferably X is O(C=O) and Y is O(C=O).

6. The compound, or the pharmaceutically acceptable salt or the stereoisomer thereof according to any one of the preceding claims, wherein X is O(C=O) and Y is O(C=O).

7. The compound, or the pharmaceutically acceptable salt or the stereoisomer thereof according to any one of the preceding claims, wherein n is selected from 6 or 8.

8. The compound, or the pharmaceutically acceptable salt or the stereoisomer thereof according to any one of the preceding claims, wherein o is selected from 6 or 8.

9. The compound, or the pharmaceutically acceptable salt or the stereoisomer thereof according to any one of the preceding claims, wherein n is 6.

10. The compound, or the pharmaceutically acceptable salt or the stereoisomer thereof according to claim 1, wherein the compound of (I) is selected from: 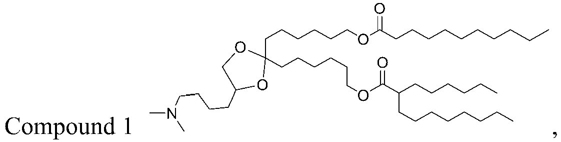 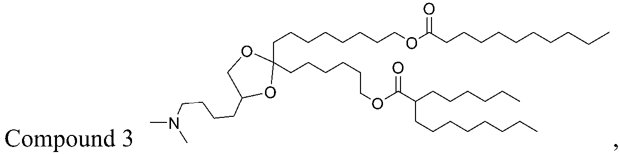 and

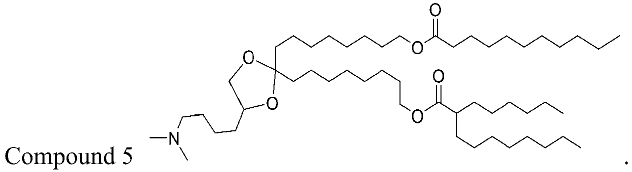

11. A lipid nanoparticle composition, comprising a lipid component, the lipid component comprising the compound, or the pharmaceutically acceptable salt or the stereoisomer thereof according to any one of claims 1 to 10.

12. The lipid nanoparticle composition according to claim 11, wherein the lipid component further comprises a phospholipid, a structural lipid, and/or a PEG lipid;
the phospholipid is preferably selected from one or more of the following compounds:
dilauroyl phosphatidylcholine (DLPC),
dimyristoyl phosphatidylcholine (DMPC),
dioleoyl phosphatidylcholine (DOPC),
dipalmitoyl phosphatidylcholine (DPPC),
distearoyl phosphatidylcholine (DSPC),
dioleoyl phosphatidylcholine (DUPC),
palmitoyl oleoyl phosphatidylcholine (POPC),
1,2-di-O-octadecyl-sn-glycero-3-phosphocholine (18:0 Diether PC),
1-oleoyl-2-cholesterylhemisuccinoyl-sn-glycero-3-phosphocholine (OChemsPC),
1-hexadecyl-sn-glycero-3-phosphocholine (C16 Lyso PC),
1,2-divinyl-sn-glycero-3-phosphocholine,
1,2-diarylacyl-sn-glycero-3-phosphocholine,
1,2-dioleoyl-SN-glycero-3-phosphoethanolamine (DOPE),
1,2-distearoyl-sn-glycero-3-phosphoethanolamine,
1,2-divinylol-sn-glycero-3-phosphoethanolamine,
1,2-divinyl-sn-glycero-3-phosphoethanolamine,
1,2-diaryl-sn-glycero-3-phosphoethanolamine,
1,2-dithiohexaenoyl-sn-glycero-3-phosphoethanolamine, and
1,2- diolyl-sn-glycero-3-phosphatidyl-(1-glycerol) sodium salt (DOPG) or sphingomyelin;
for example, the phospholipid is DOPE or DSPC;
the structural lipid is preferably selected from one or more of cholesterol, coprostanol, sitosterol, ergosterol, and stigmasterol; for example, the structural lipid is cholesterol; and/or
the PEG lipid is preferably selected from one or more of PEG-modified phosphatidylethanolamine, PEG-modified phosphatidic acid, PEG-modified ceramide, PEG-modified dialkylamine, PEG-modified diacylglycerol, or PEG-modified dialkylglycerol, and is preferably DMG-PEG2000.

13. The nanoparticle composition according to any one of claims 11 to 12, further comprising a therapeutic agent and/or a prophylactic agent, wherein the therapeutic agent and/or the prophylactic agent is selected from a vaccine, or a compound and a nucleic acid enabling triggering of an immune response, and
preferably, the nucleic acid is RNA selected from one or more of siRNA, aiRNA, miRNA, dsRNA, shRNA, or mRNA.

14. A pharmaceutical composition, comprising the nanoparticle composition according to any one of claims 11 to 13 and a pharmaceutically acceptable carrier.

15. A method for delivering a therapeutic agent and/or a prophylactic agent to a mammalian cell, comprising administering the nanoparticle composition according to any one of claims 11 to 13 or the pharmaceutical composition according to claim 14 to a subject, the administering comprising bringing a cell into contact with the nanoparticle composition or the pharmaceutical composition to deliver the therapeutic agent and/or the prophylactic agent to the cell.
